# NEUE EUROPÄISCHE PATENTSCHRIFT

(11) **EP 1 017 413 B2**
(45) Veröffentlichungstag und Bekanntmachung des Hinweises auf die Entscheidung über den Einspruch: **07.11.2007**
(45) Hinweis auf die Patenterteilung: 25.09.2002
(21) Anmeldenummer: 98950074.9
(22) Anmeldetag: 23.09.1998
(51) Int. Cl.: A61K 38/21, A61K 9/08

(54) **FLÜSSIGE INTERFERON-BETA-FORMULIERUNGEN**
LIQUID INTERFERON-BETA FORMULATIONS
FORMULATIONS LIQUIDES D'INTERFERON BETA

(30) Priorität: 23.09.1997 EP 97116562
(43) Veröffentlichungstag der Anmeldung: 12.07.2000
(60) Teilanmeldung: 02004883.1
(73) Patentinhaber: Rentschler Biotechnologie GmbH, 88471 Laupheim (DE)
(72) Erfinder: TSCHÖPE, Michael, D-88400 Biberach (DE); SIKLOSI, Thomas, D-88487 Walpertshofen (DE); SCHROEDER, Peter, D-88471 Laupheim (DE); HOFER, Hans, D-88487 Walpertshofen (DE)
(74) Vertreter: Weiss, Wolfgang
(86) Internationale Anmeldenummer: PCT/EP1998/006065
(87) Internationale Veröffentlichungsnummer: WO 1999/015193

(56) Entgegenhaltungen:
- EP-A- 0 082 481
- EP-A- 0 284 249
- EP-A1- 0 374 257
- EP-A1- 0 529 300
- WO-A-92/15614
- WO-A1-98/28007
- JP-A- 59 181 224
- US-A- 5 358 708

### SEQUENZPROTOKOLL

### (1) ALLGEMEINE ANGABEN:

(i) ANMELDER:
   (A) NAME: Dr.Rentschler Biotechnologie GmbH
   (B) STRASSE: Erwin-Rentschler-Str. 21
   (C) ORT: Laupheim
   (E) LAND: Deutschland
   (F) POSTLEITZAHL: D-88471
(ii) BEZEICHNUNG DER ERFINDUNG: Flüssige Interferon-β Formulierungen
(iii) ANZAHL DER SEQUENZEN: 14
(iv) COMPUTER-LESBARE FASSUNG:
   (A) DATENTRÄGER: Floppy disk
   (B) COMPUTER: IBM PC compatible
   (C) BETRIEBSSYSTEM: PC-DOS/MS-DOS
   (D) SOFTWARE: Patent In Release #1.0, Version #1.30 (EPA)

### (2) ANGABEN ZU SEQ ID NO: 1:

(i) SEQUENZKENNZEICHEN:
   (A) LÄNGE: 7 Aminosäuren
   (B) ART: Aminosäure
   (C) STRANGFORM: Einzelstrang
   (D) TOPOLOGIE: linear
(ii) ART DES MOLEKÜLS: Peptid
(viii) POSITION IM GENOM:
   (B) KARTENPOSITION: 109-115
(xi) SEQUENZBESCHREIBUNG: SEQ ID NO: 1:
   Glu Asp Phe Thr Arg Gly Lys
   1               5

### (2) ANGABEN ZU SEQ ID NO: 2:

(i) SEQUENZKENNZEICHEN:
   (A) LÄNGE: 6 Aminosäuren
   (B) ART: Aminosäure
   (C) STRANGFORM: Einzelstrang
   (D) TOPOLOGIE: linear
(ii) ART DES MOLEKÜLS: Peptid
(viii) POSITION IM GENOM:
   (B) KARTENPOSITION: 100-105
(xi) SEQUENZBESCHREIBUNG: SEQ ID NO: 2:
   Thr Val Leu Glu Glu Lys
   1               5

### (2) ANGABEN ZU SEQ ID NO: 3:

(i) SEQUENZKENNZEICHEN:
   (A) LÄNGE: 7 Aminosäuren
   (B) ART: Aminosäure
   (C) STRANGFORM: Einzelstrang
   (D) TOPOLOGIE: linear
(ii) ART DES MOLEKÜLS: Peptid
(viii) POSITION IM GENOM:
   (B) KARTENPOSITION: 46-52
(xi) SEQUENZBESCHREIBUNG: SEQ ID NO: 3:
   Gln Leu Gln Gln Phe Gln Lys
   1               5

### (2) ANGABEN ZU SEQ ID NO: 4:

(i) SEQUENZKENNZEICHEN:
   (A) LÄNGE: 8 Aminosäuren
   (B) ART: Aminosäure
   (C) STRANGFORM: Einzelstrang
   (D) TOPOLOGIE: linear
(ii) ART DES MOLEKÜLS: Peptid
(viii) POSITION IM GENOM:
   (B) KARTENPOSITION: 116-123
(ix) MERKMAL:
   (A) NAME/SCHLÜSSEL: Modified-site
   (B) LAGE:2
   (D) SONSTIGE ANGABEN:/product= "Xaa = Met(oxidiert)"
(xi) SEQUENZBESCHREIBUNG: SEQ ID NO: 4:
   Leu Xaa Ser Ser Leu His Leu Lys
   1               5

### (2) ANGABEN ZU SEQ ID NO: 5:

(i) SEQUENZKENNZEICHEN:
   (A) LÄNGE: 8 Aminosäuren
   (B) ART: Aminosäure
   (C) STRANGFORM: Einzelstrang
   (D) TOPOLOGIE: linear
(ii) ART DES MOLEKÜLS: Peptid
(viii) POSITION IM GENOM:
   (B) KARTENPOSITION: 116-123
(xi) SEQUENZBESCHREIBUNG: SEQ ID NO: 5:
   Leu Met Ser Ser Leu His Leu Lys
   1               5

### (2) ANGABEN ZU SEQ ID NO: 6:

(i) SEQUENZKENNZEICHEN:
   (A) LÄNGE: 12 Aminosäuren
   (B) ART: Aminosäure
   (C) STRANGFORM: Einzelstrang
   (D) TOPOLOGIE: linear
(ii) ART DES MOLEKÜLS: Peptid
(viii) POSITION IM GENOM:
   (B) KARTENPOSITION: 34-45
(ix) MERKMAL:
   (A) NAME/SCHLÜSSEL: Modified-site
   (B) LAGE:3
   (D) SONSTIGE ANGABEN:/product= "Xaa = Met(oxi diert)"
(xi) SEQUENZBESCHREIBUNG: SEQ ID NO: 6:
   Asp Arg Xaa Asn Phe Asp Ile Pro Glu Glu Ile Lys
   1               5                   10

### (2) ANGABEN ZU SEQ ID NO: 7:

(i) SEQUENZKENNZEICHEN:
   (A) LÄNGE: 11 Aminosäuren
   (B) ART: Aminosäure
   (C) STRANGFORM: Einzelstrang
   (D) TOPOLOGIE: linear
(ii) ART DES MOLEKÜLS: Peptid
(viii) POSITION IM GENOM:
   (B) KARTENPOSITION: 124-134
(xi) SEQUENZBESCHREIBUNG: SEQ ID NO: 7:
   Arg Tyr Tyr Gly Arg Ile Leu His Tyr Leu Lys
   1               5                   10

### (2) ANGABEN ZU SEQ ID NO: 8:

(i) SEQUENZKENNZEICHEN:
   (A) LÄNGE: 12 Aminosäuren
   (B) ART: Aminosäure
   (C) STRANGFORM: Einzelstrang
   (D) TOPOLOGIE: linear
(ii) ART DES MOLEKÜLS: Peptid
(viii) POSITION IM GENOM:
   (B) KARTENPOSITION: 34-45
(xi) SEQUENZBESCHREIBUNG: SEQ ID NO: 8:
   Asp Arg Met Asn Phe Asp Ile Pro Glu Glu Ile Lys
   1               5                   10

### (2) ANGABEN ZU SEQ ID NO: 9:

(i) SEQUENZKENNZEICHEN:
   (A) LÄNGE: 14 Aminosäuren
   (B) ART: Aminosäure
   (C) STRANGFORM: Einzelstrang
   (D) TOPOLOGIE: linear
(ii) ART DES MOLEKÜLS: Peptid
(viii) POSITION IM GENOM:
   (B) KARTENPOSITION: 20-33
(xi) SEQUENZBESCHREIBUNG: SEQ ID NO: 9:
   Leu Leu Trp Gln Leu Asn Gly Arg Leu Glu Tyr Cys Leu Lys
   1               5                   10

### (2) ANGABEN ZU SEQ ID NO: 10:

(i) SEQUENZKENNZEICHEN:
   (A) LÄNGE: 19 Aminosäuren
   (B) ART: Aminosäure
   (C) STRANGFORM: Einzelstrang
   (D) TOPOLOGIE: linear
(ii) ART DES MOLEKÜLS: Peptid
(viii) POSITION IM GENOM:
   (B) KARTENPOSITION: 1-19
(ix) MERKMAL:
   (A) NAME/SCHLÜSSEL: Modified-site
   (B) LAGE:1
   (D) SONSTIGE ANGABEN:/product= "Xaa = Met(oxidiert)"
(xi) SEQUENZBESCHREIBUNG: SEQ ID NO: 10:
   Xaa Ser Tyr Asn Leu Leu Gly Phe Leu Gln Arg Ser Ser Asn Phe Gln
   1               5                   10                  15
   Cys Gln Lys

### (2) ANGABEN ZU SEQ ID NO: 11:

(i) SEQUENZKENNZEICHEN:
   (A) LÄNGE: 19 Aminosäuren
   (B) ART: Aminosäure
   (C) STRANGFORM: Einzelstrang
   (D) TOPOLOGIE: linear
(ii) ART DES MOLEKÜLS: Peptid
(viii) POSITION IM GENOM:
   (B) KARTENPOSITION: 1-19
(xi) SEQUENZBESCHREIBUNG: SEQ ID NO: 11:
   Met Ser Tyr Asn Leu Leu Gly Phe Leu Gln Arg Ser Ser Asn Phe Gln
   1               5                   10                  15
   Cys Gln Lys

### (2) ANGABEN ZU SEQ ID NO: 12:

(i) SEQUENZKENNZEICHEN:
   (A) LÄNGE: 30 Aminosäuren
   (B) ART: Aminosäure
   (C) STRANGFORM: Einzelstrang
   (D) TOPOLOGIE: linear
(ii) ART DES MOLEKÜLS: Peptid
(viii) POSITION IM GENOM:
   (B) KARTENPOSITION: 137-166
(xi) SEQUENZBESCHREIBUNG: SEQ ID NO: 12:
   Glu Tyr Ser His Cys Ala Trp Thr Ile Val Arg Val Glu Ile Leu Arg
   1               5                   10                  15
   Asn Phe Tyr Phe Ile Asn Arg Leu Thr Gly Tyr Leu Arg Asn
               20                  25                  30

### (2) ANGABEN ZU SEQ ID NO: 13:

(i) SEQUENZKENNZEICHEN:
   (A) LÄNGE: 47 Aminosäuren
   (B) ART: Aminosäure
   (C) STRANGFORM: Einzelstrang
   (D) TOPOLOGIE: linear
(ii) ART DES MOLEKÜLS: Peptid
(viii) POSITION IM GENOM:
   (B) KARTENPOSITION: 53-99
(ix) MERKMAL:
   (A) NAME/SCHLÜSSEL: Modified-site
   (B) LAGE:10
   (D) SONSTIGE ANGABEN:/product= "Xaa = Met(oxidiert)"
(xi) SEQUENZBESCHREIBUNG: SEQ ID NO: 13:
   Glu Asp Ala Ala Leu Thr Ile Tyr Glu Xaa Leu Gln Asn Ile Phe Ala
   1               5                   10                  15
   Ile Phe Arg Gln Asp Ser Ser Ser Thr Gly Trp Asn Glu Thr Ile Val
               20                  25                  30
   Glu Asn Leu Leu Ala Asn Val Tyr His Gln Ile Asn His Leu Lys
           35                  40                  45

### (2) ANGABEN ZU SEQ ID NO: 14:

(i) SEQUENZKENNZEICHEN:
   (A) LÄNGE: 47 Aminosäuren
   (B) ART: Aminosäure
   (C) STRANGFORM: Einzelstrang
   (D) TOPOLOGIE: linear
(ii) ART DES MOLEKÜLS: Peptid
(viii) POSITION IM GENOM:
   (B) KARTENPOSITION: 53-99
(xi) SEQUENZBESCHREIBUNG: SEQ ID NO: 14:
   Glu Asp Ala Ala Leu Thr Ile Tyr Glu Met Leu Gln Asn Ile Phe Ala
   1               5                   10                  15
   Ile Phe Arg Gln Asp Ser Ser Ser Thr Gly Trp Asn Glu Thr Ile Val
               20                  25                  30
   Glu Asn Leu Leu Ala Asn Val Tyr His Gln Ile Asn His Leu Lys
           35                  40                  45

## Beschreibung

Die vorliegende Erfindung betrifft flüssige Formulierungen von humanem Interferon-β. Die Formulierungen sind dadurch gekennzeichnet, daß sie einen pH-Wert im schwach sauren bis neutralen Bereich zwischen 5 und 8 aufweisen sowie eine hohe Stabilität des Interferon-β in Lösung unter Beibehalt der molekularen Integrität gegeben ist.

Natürlich vorkommende Interferone sind speziesspezifische Proteine, teilweise Glykoproteine, die durch unterschiedliche Zellen des Körpers nach Induktion mit Viren, doppelsträngiger RNA, anderen Polynukleotiden sowie Antigenen erzeugt werden. Interferone besitzen zahlreiche biologische Aktivitäten wie z.B. antivirale, antiproliferative sowie immunmodulatorische Eigenschaften. Es sind mindestens 3 unterschiedliche Typen humaner Interferone identifiziert worden, welche durch Leukozyten, Lymphozyten, Fibroblasten sowie Zellen des Immunsystems produziert werden und als α-, β-, γ-Interferone bezeichnet werden. Einzelne Interferontypen können weiterhin in zahlreiche Subtypen unterteilt werden.

Natives, menschliches Interferon-β kann durch Superinduktion humaner Fibroblastenzellkulturen mit Poly-IC sowie anschließende Isolierung und Reinigung des Interferon β durch chromatographische und elektrophoretische Techniken industriell hergestellt werden. Proteine oder Polypeptide, welche dem natürlichen Interferon-β vergleichbare Eigenschaften aufweisen, können auch durch rekombinante DNA-Technologien hergestellt werden (EP-A-0 028 033; EP-A- 0 041 313; EP-A-0 070 906; EP-A-0 287 075; Chernajovsky et al. (1984) DNA 3, 297-308; McCormick et al. (1984) Mol. Cell. Biol. 4, 166-172). Dabei kann rekombinantes humanes Interferon-β sowohl in eukaryontischen Zellen (z.B. CHO-Zellen) als auch von prokaryontischen Zellen (z.B. E.coli) produziert werden. Die entsprechenden Interferone werden als Interferon-β-1a bzw. Interferon-β-1b bezeichnet. Im Gegensatz zu Interferon-β-1b ist Interferon-β-1a glykosiliert (Goodkin (1994) Lancet 344, 1057-1060).

Der therapeutische Einsatz von Interferon-β setzt voraus, daß es in eine galenische Zubereitung gebracht wird, die das Protein über längere Zeit unter Erhaltung der molekularen Integrität lagerfähig macht. Interferon-β ist instabil und unterliegt unterschiedlichen Abbaureaktionen. Hierzu gehören insbesondere die Spaltung von Peptidbindungen, Deamidierung, Oxidation des Methionins zu Methioninsulfid, Disulfidaustausch sowie Veränderungen der Zuckerseitenkette bis hin zur Deglycosilierung.

Aufgrund des therapeutischen Nutzens von Interferonen sind in den vergangenen Jahren eine Reihe von Formulierungen entwickelt worden, die jedoch alle gewisse Nachteile aufweisen. Das US-Patent Nr. 4,647,454 (Inter-Yeda Ltd.) beschreibt eine Formulierung von Fibroblasten Interferon-β, die durch Zusatz von Polyvinylpyrrolidon (PVP) im stark sauren Bereich (pH 3,5) stabilisiert werden kann. Weitere bevorzugte Hilfsstoffe sind Mannitol, Humanserumalbumin sowie Acetatpuffer. Die Formulierung wird gefriergetrocknet und bei 4°C aufbewahrt.

Die japanische Patentschrift 59 181 224 (Sumitomo Chemical Co.) beschreibt eine wässrige Lösung von Interferonen, bei welcher polare Aminosäuren wie Arginin, Asparagin, Glutaminsäure, Glutamin, Histidin, Lysin, Serin sowie Threonin bzw. deren Natriumsalze zusammen mit Humanserumalbumin zur Stabilisierung der Interferone eingesetzt werden.

Die internationale Patentanmeldung WO 95/31213 (Applied Research Systems ARS Holding) beschreibt eine flüssige Formulierung für Interferon-β, die durch Zusatz eines Polyols, bevorzugt Mannitol, und eines nichtreduzierenden Zuckers oder einer Aminosäure stabilisiert wird. Die Formulierung enthält weiterhin einen Puffer (Acetatpuffer pH 3,0 bis 4,0) sowie Humanserumalbumin. Während Rezepturen mit einem pH-Wert zwischen 5 und 6 einen sofortigen Verlust an biologischer Aktivität zeigten, sind die in der Patentschrift bevorzugten Rezepturen bei pH-Werten von 3,0 sowie 4,0 hinreichend stabil. Die Aussage der Stabilität bezieht sich außerdem nur auf die biologische Aktivität der Formulierung, nicht aber auf die molekulare Integrität des Wirkstoffs.

Die europäische Patentanmeldung EP 0 215 658 (Cetus Corp.) beschreibt eine Formulierung für rekombinantes Interferon-β, in welcher die biologisch aktive Verbindung in einem wässrigen Medium bei einem pH-Wert zwischen 2 und 4 unter Zusatz von Stabilisatoren wie Humanserumalbumin oder Humanplasmaproteinfraktionen sowie gegebenenfalls Dextrose gelöst wird. Eine weitere Patentanmeldung der Cetus Corp. (WO 89/05158) beschreibt eine Formulierung für Interferon-β, die bei einem pH-Wert zwischen 2 und 4 als Stabilisatoren entweder Glycerin oder Polyethylenglycopolymere mit einem durchschnittlichen Molekulargewicht zwischen 190 bis 1.600 Dalton einsetzen. Als geeignete Pufferkomponenten werden Glycin, Phosphorsäure sowie Zitronensäure genannt.

Die europäische Patentanmeldung EP 0 217 645 (Cetus Corp.) beschreibt pharmazeutische Zubereitungen mit IL-2 oder Interferon-β, die in einem Trägermedium bei pH 7 bis 8 gelöst und unter Zusatz von Natriumlaurat als oberflächenaktive Verbindung stabilisiert sind. Darüber hinaus wird zur Stabilisierung dieser Zubereitungen auch SDS als weitere ionische oberflächenaktive Verbindung benötigt.

Das europäische Patent EP 0 270 799 (Cetus Oncology Corp.) beschreibt eine Formulierung für nichtglycosiliertes rekombinantes Interferon-β in einem inerten Trägermedium auf Wasserbasis, das als Stabilisator nichtionische polymere Detergenzien enthält.

Die europäische Patentanmeldung EP 0 529 300 (Rentschler Biotechnolgie GmbH) beschreibt flüssige Interferon-β-Formulierungen, die eine Konzentration von 30 bzw. 70 MU/ml rekombinantes IFN-β, Natriumchlorid und Imidazol- bzw. Natriumphosphatpuffer enthalten sowie einen pH-Wert von 7,5 aufweisen (Beispiel 3). Diese Formulierungen sind für 4 Wochen bei einer Lagertemperatur von 25°C hinsichtlich ihrer biologischen Aktivität stabil. Diese Zusammensetzungen haben jedoch den Nachteil, daß die verwendete Konzentration von Interferon-β (≥ 30 MU/ml) für praktische Anwendungen zu hoch ist. Darüber hinaus findet sich in EP-A-0 529 300 keinerlei Hinweis, daß durch Zusatz von Humanserumalbumin die Stabilität von flüssigen Interferon-β Formulierungen verringert wird. Im Gegenteil wird der Zusatz von Humanserumalbumin als bevorzugt bezeichnet.

Die internationale Patentanmeldung WO 98/28007 (Biogen, Inc.) beschreibt die Verwendung von sauren Aminosäuren, Arginin und Glycin in Interferon enthaltenen Formulierungen als stabilisierende Agentien.

Neben Formulierungen für Interferon-β sind auch pharmazeutische Darreichungsformen mit Interferon-α beschrieben. Die europäische Patentschrift 0 082 481 (Schering Corp.) offenbart eine zur Gefriertrocknung bestimmte wässrige Formulierung, die neben einem Phosphatpuffer und Glycin Humanserumalbumin enthält. Als weiterer optionaier Bestandteil wird Alanin genannt. Der pH-Wert der Lösung nach Rekonstitution liegt zwischen 7,0 und 7,4. Eine weitere Patentanmeldung der Schering Corp. (WO 96/11018) offenbart stabile wässrige Lösungen im Interferon-α, die bei einem pH-Wert zwischen 4,5 und 7,1 Chelatbildner (NaEDTA oder Zitronensäure), eine oberflächenaktive Verbindung (Polysorbat 80), ein Isotonisierungsmittel (Natriumchlorid) sowie geeignete Konservierungsmittel wie Methylparaben, Propylparaben, m-Kresol oder Phenol beinhalten. Die offenbarten wässrigen Formulierungen erweisen sich bezüglich der biologischen Aktivität (Standardmethode der Hemmung des zytopatischen Effekts (CPE) eines Virus wie beschrieben bei W.P. Protzman in J. Clinical Microbiology, 1985, 22, S. 596-599) bei 25°C für 6 Monate als stabil (biologische Aktivität >90% der Ausgangsaktivität). Eine parallel durchgeführte Bestimmung des Proteingehalts mittels HPLC weist nach 6 Monaten bei 25°C jedoch bereits Gehaltsverluste zwischen 20,2 (Tab.3) oder 32,5% (Tab. 4) aus.

EP-A-0 736 303 (Hoffmann-LaRoche AG) offenbart wäßrige Interferon-α-Zusammensetzungen, die neben einem Interferon-α ein nichtionisches Detergens, einen Puffer zur Einstellung des pH-Bereiches zwischen 4,5 und 5,5, Benzylalkohol und gegebenenfalls ein isotonisierendes Mittel umfassen. Bei Bestimmung mittels HPLC wird nach dreimonatiger Lagerung bei 25°C und einer Ausgangskonzentration von 18 MU Interferon-α2a ein Restgehalt von 84,5 % ermittelt, bei Weglassen des Stabilisators Benzylalkohol sinkt dieser Wert auf 62,8 %.

EP-A-0 641 567 (Ciba Geigy AG) beschreibt pharmazeutische Zusammensetzungen, die Hybrid-Interferon-α und als Stabilisator einen Puffer mit einem pH-Wert zwischen 3.0 und 5.0 enthalten.

Das US-Patent 5,358,708 (Schering Corp.) beschreibt wässrige Formulierungen von Interferon-α, die als Stabilisator Methionin, Histidin oder Mischungen davon enthalten. Nach zweiwöchiger Lagerung einer Interferon-α Lösung bei 40°C wird eine Abnahme des Wirkstoffgehalts um 20 % gefunden.

Die oben aufgeführten Formulierungen für Interferone sind aus heutiger Sicht mit Nachteilen behaftet, da z.B. auf Zusatz von Humanserumalbumin zur Stabilisierung von Proteinen aus Gründen der gestiegenen Anforderungen an die Sicherheit vor Viruskontaminationen durch Blutspender verzichtet werden sollte. Desweiteren ist für eine Vielzahl der oben beschriebenen Formulierungen ein Zusatz von Aminosäuren und/oder eine Gefriertrocknung erforderlich. Gefriergetrocknete Produkte sind jedoch in ihrer Herstellung sehr aufwendig und entsprechend teuer und erfordern durch die Notwendigkeit zur Rekonstitution einen zusätzlichen Arbeitsschritt, der insbesondere für Patienten mit eingeschränkter Motorik oftmals nur sehr schwer zu vollziehen ist. Eine Reihe von Rezepturen weisen unphysiologische pH-Werte unterhalb von 5,0 auf. Obschon derartige Werte nicht gänzlich unüblich sind (siehe auch S. Sweetana und N.J. Aders, Journal of Pharmaceutical Sciences and Technology, 1996, 50: 330-342), muß bei intramuskulärer oder subkutaner Applikation mit schmerzhaften Irritationen gerechnet werden. Die Verwendung von oberflächenaktiven Verbindungen, wie Polysorbat 80, ist entsprechend Sweetana und Akers zwar zulässig, es sind jedoch eine Reihe von Nebenwirkungen insbesondere auch bei Kindern und Neugeborenen beschrieben, die den Einsatz derartiger Hilfsstoffe in Frage stellen. Über die Toxizität von oberflächenaktiven Verbindungen wird zusammenfassend bei Attwood und Florence (Surfactant Systems, their Chemistry, Pharmacy and Biology, Chapman and Hall; London, 1983) berichtet. Eine Übersicht über die Pharmakologie von Polysorbat 80 befindet sich bei R.K. Varma et al. (Arzneim.-Forsch./ Drug Res. 35, 1985, 804-808).

Aufgrund der oben genannten Nachteile, sollte eine optimale Formulierung für Interferon-β folgende Eigenschaften in sich vereinigen:
- Erhalt der biologischen Aktivität über den Lagerzeitraum,
- Erhalt der molekularen Integrität des Wirkstoffmoleküls über den Lagerzeitraum,
- Flüssige Formulierung, Verzicht auf eine teure Gefriertrocknung sowie eine zusätzliche Rekonstitution,
- Verzicht auf risikobehaftete Hilfsstoffe wie Humanserumalbumin oder oberflächenaktive Verbindungen (Detergenzien),
- pH-Wert im neutralen bis schwach sauren Bereich.

Sämtliche Forderungen werden durch die im nachfolgenden Abschnitt genauer beschriebene Erfindung erfüllt.

Überraschenderweise wurde eine Rezepturzusammensetzung gefunden, welche die molekulare Integrität von Interferon-β in flüssiger Form über einen langen Zeitraum in einem physiologischen pH-Bereich zwischen 5 und 8, bevorzugt zwischen 5,5 und 8 sicherstellt, ohne auf die als nachteilig bekannten Hilfsstoffe des Standes der Technik zurückgreifen zu müssen.

Ein erster Aspekt der Erfindung ist eine flüssige pharmazeutische Formulierung, die humanes glykosiliertes IFN-β als Wirkstoff, einen Puffer zur Einstellung eines pH-Werts zwischen 5 und 8, bevorzugt zwischen 5,5 und 8 und Methionin enthält, die frei von Humanserumalbumin ist, mit der Maßgabe, dass die Formulierung keine sauren Aminosäuren, Arginin und Glycin in einem Gewichtsanteil zwischen 0,3-5% enthält, und eine Langzeitstabilität der biologischen Aktivität (in vitro) von mindestens 80% der Ausgangsaktivität nach Lagerung bei 25°C für 3 Monate aufweist.

Die Messung der Langzeitstabilität von flüssigen pharmazeutischen Formulierungen erfolgte bei 25°C. Die Temperatur von 25°C wurde gewählt, um auf der einen Seite eine Beschleunigung von Abbaureaktionen zu bewirken, auf der anderen Seite jedoch keine durch überhöhte Temperaturen bewirkten Artefakte hervorzurufen. Geeignete analytische Methoden zur Bestimmung der Stabilität von Interferon-β sind in den Übersichtsartikeln von J. Geigert (J. Parent. Sci. Technol. 43 (1989), 220-224) oder M.C. Manning, K. Patel und R.T. Borchardt (Pharm. Res. 6 (1989), 903-918) beschrieben.

Die Messung der biologischen Aktivität nach der jeweils gewählten Aufbewahrungsdauer erfolgte durch die Standardmethode der Inhibierung des zytopathischen Effekts eines Virus. Eine genaue Beschreibung der verwendeten Testmethode findet sich bei Stewart, W.E. II (1981): The Interferon System (Second, enlarged Edition), Springer-Verlag: Wien, New York; Grossberg, S.E. et al. (1984), Assay of Interferons. In: Came, P.E., Carter W.A (eds) Interferons and their Applications, Springer-Verlag: Berlin, Heidelberg, New York, Tokyo, pp. 23-43. Eine erfindungsgemäße Formulierung weist nach dreimonatiger Aufbewahrung bei 25°C eine biologische Aktivität von mindestens 80%, vorzugsweise von mindestens 85% und besonders bevorzugt von mindestens 90% der Ausgangsaktivität auf.

Vorzugsweise besitzt eine erfindungsgemäße Formulierung nach sechsmonatiger Aufbewahrung bei 25°C eine biologische Aktivität von mindestens 80% und vorzugsweise von mindestens 85% der Ausgangsaktivität.

Auch bei einer Aufbewahrung bei höheren Temperaturen, z.B. 37°C, weisen die erfindungsgemäßen Formulierungen eine überraschend hohe Langzeitstabilität der biologischen Aktivität auf. So wird nach einer einmonatigen Aufbewahrung bei 37°C eine biologische Aktivität von mindestens 70% und vorzugsweise von mindestens 80% der Ausgangsaktivität gefunden.

Die erfindungsgemäßen flüssigen pharmazeutischen Formulierungen sind frei von Humanserumalbumin und besonders bevorzugt - abgesehen vom Wirkstoff - frei von humanen oder tierischen Polypeptiden insbesondere von Serumproteinen. Weiterhin ist bevorzugt, daß die erfindungsgemäße flüssige pharmazeutische Formulierung frei von oberflächenaktiven Mitteln, insbesondere frei von ionischen Detergenzien oder/und nichtionischen Tensiden ist.

Die erfindungsgemäßen Formulierungen enthalten als Wirkstoff ein humanes glykosiliertes Interferon-β, d.h. ein Polypeptid, welches biologische oder/und immunologische Eigenschaften von natürlichem humanem Interferon-β aufweist und ein natürlich vorkommendes oder rekombinantes Interferon-β sein kann. Vorzugsweise enthält die Formulierung ein rekombinantes Interferon-β aus CHO-Zellen. Am meisten bevorzugt werden Interferon-β Spezies verwendet, wie sie aus der Zellinie BIC 8622 (ECACC 87 04 03 01) erhältlich sind und beispielsweise in EP-B-0 287 075 und EP-A-0 529 300 beschrieben sind.

Der Wirkstoff liegt in den erfindungsgemäßen Formulierungen vorzugsweise in einer Konzentration bis zu 25 MU/ml vor. Bevorzugt ist jedoch eine Dosierung im Bereich von 1 bis 25 MU/ml, besonders bevorzugt von 3 bis 20 MU/ml, am meisten bevorzugt 3 bis 10 MU/ml. Diese Dosierungsbereiche erlauben eine unmittelbare Anwendung ohne weitere Verdünnung in Verbindung mit einer besonders guten Stabilität bei erhöhter Temperatur.

Ein weiteres bevorzugtes Merkmal der erfindungsgemäßen flüssigen pharmazeutischen Formulierung ist, daß sie eine chemische Integrität nach Lagerung bei 25°C für 3 Monate und vorzugsweise für 6 Monate aufweist, d.h. daß sie beständig gegenüber Peptidspaltung, Oxidation und Deglykosilierung ist. Die Messung der chemischen Integrität erfolgt durch Peptidmapping, Westernblot sowie Glykosilierungsanalyse. Als chemisch stabil im Zusammenhang mit der vorliegenden Erfindung sind Zusammensetzungen zu betrachten, bei welchen das Interferon-β im Anschluß an die Formulierung mindestens 85%, vorzugsweise mindestens 90% der chemischen Integrität bei den gewählten Lagerungsbedingungen beibehält.

Ein weiteres bevorzugtes Merkmal der erfindungsgemäßen flüssigen pharmazeutischen Formulierungen ist eine physikalische Integrität nach Lagerung bei 25°C für 3 Monate und vorzugsweise für 6 Monate. Dabei wird die physikalische Integrität durch Messung der Transmission bei 420 nm sowie durch visuelle Betrachtung der Lösungen gemessen. Als physikalisch stabil sind diejenigen Lösungen anzusehen, deren Transmission über 90%, vorzugsweise über 93% bei den gewählten Lagerungsbedingungen liegt, und bei welchen keine Trübung bei visueller Betrachtung festgestellt werden kann.

Durch die vorliegende Erfindung können überraschenderweise flüssige Formulierungen von Interferon-β bereitgestellt werden, die über einen langen Zeitraum biologisch, chemisch und physikalisch stabil sowie frei von unerwünschten Inhaltsstoffen wie etwa Humanserumalbumin oder oberflächenaktiven Mitteln sind. Die erfindungsgemäßen Formulierungen enthalten neben dem Wirkstoff einen Puffer, der vorzugsweise in einer Konzentration von 10 mmol/l bis 1 mol/l, besonders bevorzugt in einer Konzentration von 20 mmol/l bis 200 mmol/l, z.B. ca. 50 mmol/l bis 100 mmol/l vorliegt und dazu dient, den pH-Wert der Formulierung im Bereich von 5 bis 8, bevorzugt von 5,5 bis 8 und stärker bevorzugt zwischen 6 und 7,4 zu halten. Besonders bevorzugt ist ein pH-Bereich zwischen 6 und 7,2 und am meisten bevorzugt zwischen 6,2 und 6,8, da hier eine besonders hohe Stabilität unter Beibehalt der molekularen Integrität erreicht wird. Der Puffer wird aus pharmazeutisch akzeptablen Puffern ausgewählt, z.B. Borat-, Succinat-, L-Malat-, TRIS-, Salicylat-, Glycylglycin-, Triethanolamin-, Isocitrat-, Maleat-, Phosphat-, Citrat- und Acetatpuffer oder Mischungen davon. Bevorzugt verwendet man Phosphat-, Citrat- und Acetatpuffer oder Mischungen davon, besonders bevorzugt Phosphat/Citratpuffer.

Die erfindungsgemäße Formulierung kann neben dem Wirkstoff und dem Puffer weitere physiologisch verträgliche Hilfsstoffe enthalten, beispielsweise Hilfsstoffe zur Anpassung der Tonizität an die Tonizität des Blutes oder Gewebe, z.B. nichtreduzierende Zucker, Zuckeralkohole wie Mannit, Sorbit, Xylit oder Glycerin. Der erfindungsgemäßen Formulierung wird die Aminosäure Methionin zur weiteren Erhöhung der chemischen Stabilität zugesetzt. Die Konzentration von Methionin liegt vorzugsweise im Bereich von 0,1 bis 4 mmol/l. Besonders bevorzugt ist eine Konzentration vom 2 mmol/l. Weiterhin kann die Zusammensetzung Verdickungsmittel zur Viskositätserhöhung, z.B. für ophthalmologische Zwecke, enthalten. Beispiele für geeignete Verdickungsmittel sind ophthalmologisch geeignete Polymere, z.B. Carbopol, Methylcellulose, Carboxymethylcellulose etc.

Darüber hinaus kann die erfindungsgemäße Zusammensetzung auch Konservierungsmittel enthalten. Für ophthalmologische Zwecke kann beispielsweise Thiomersal in einer Menge von 0,001 bis 0,004% (Gewicht/Volumen) zum Einsatz kommen.

Die Erfindung betrifft weiterhin pharmazeutische Präparate, die eine flüssige Interferon-β enthaltende Formulierung wie oben beschrieben enthalten. Die-se pharmazeutischen Präparate sind insbesondere für die orale, parenterale oder ophthalmologische Applikation geeignet. Die Formulierungen liegen vorzugsweise in Einzeldosen von 1 bis 25 MU IFN-β vor. Weiterhin betrifft die Erfindung ein Verfahren zur Herstellung derartiger pharmazeutischer Präparate, wobei man eine erfindungsgemäße Formulierung und gegebenenfalls weitere galenisch notwendige Hilfsstoffe zubereitet und in eine geeignete Darreichungsform bringt.

Die erfindungsgemäße Formulierung kann in geeigneten, gewaschenen sowie sterilisierten Glasvials (hydrolytische Klasse 1) mit pharmazeutisch akzeptablen Gummistopfen gelagert werden.

Desweiteren können erfindungsgemäße Formulierungen auch aseptisch in Fertigspritzen oder aber in Karpulen zum Einsatz in Selbstinjektionssystemen abgefüllt und eingesetzt werden. Die wässrigen Lösungen können - obwohl dies nicht bevorzugt ist - durch Zusatz weiterer, dem Fachmann bekannter Hilfsstoffe gefriergetrocknet werden und stehen dann nach Rekonstitution in flüssiger Form zur Verfügung.

Unter Zusatz von geeigneten Konservierungsmitteln können flüssige Mehrfachdosisarzneiformen sowie Augentropfenlösungen und Tropflösungen zur oralen Applikation hergestellt werden.

Die zur Herstellung der entsprechenden Darreichungsformen noch zusätzlich benötigten Hilfsstoffe sind dem Fachmann bekannt.

Schließlich betrifft die Erfindung ein Verfahren zur Verbesserung der Haltbarkeit einer flüssigen Formulierung, die humanes glykosiliertes Interferon-β als Wirkstoff und einen Puffer zur Einstellung eines pH-Werts von 5 bis 8, bevorzugt von 5,5 bis 8 enthält, dadurch gekennzeichnet, daß man eine Formulierung ohne Humanserumalbumin und mit Methionin verwendet mit der Maßgabe, daß die Formulierung keine sauren Aminosäuren, Arginin oder Glycin in einem Gewichtsanteil zwischen 0,3 bis 5% enthält. Die Verbesserung der Haltbarkeit umfaßt eine Verbesserung der Langzeitstabilität der biologischen Aktivität (in vitro), der chemischen Integrität oder/und der physikalischen Integrität wie vorstehend angegeben.

Weiterhin wird die Erfindung durch die nachfolgenden Beispiele und Vergleichsbeispiele erläutert.

### Beispiele

In allen Beispielen wurde ein aus CHO-Zellen gewonnenes Interferon-β verwendet.

### 1. Langzeitstabilität von flüssigen Interferon-β Formulierunqen bei 25°C

Es wurden folgende Formulierungen getestet:
- Formulierung 1:: 50 mmol/l Natriumcitrat pH 5,0
- Formulierung 2:: 50 mmol/l Natriumcitrat, 50 mmol/l, Natriumphosphat pH 7,0, 15 mg/ml Humanserumalbumin, 2 mmol/l Methionin, 50 mg/ml Glycerin
- Formulierung 3:: 50 mmol/l Natriumcitrat, 50 mmol/l Natriumphosphat pH 7,0, 50 mg/ml Glycerin, 2 mmol/l Methionin
- Formulierung 4:: 50 mmol/l Natriumcitrat, 50 mmol/l Natriumphosphat pH 7,0, 2 mmol/l Methionin
- Formulierung 5:: 50 mmol/l Natriumcitrat, 50 mmol/l Natriumphosphat pH 7,0
- Formulierung 17:: 70 mmol/l Natriumcitrat, 50 mmol/l Natriumphosphat, 2 mmol/l Methionin, pH 6,5

Die Formulierungen wurden auf einen Gehalt von ca. 10 bis 15 MU/ml (d.h. 10 bis 15 x 10⁶ I.E./ml) verdünnt.

Die Formulierungen wurden mit Ausnahme von Formulierung 17 (s.u.) in Glasvials der hydrolytischen Klasse 1 (DIN 2R Vials), die mit handelsüblichen Chlorbuthylgummistopfen verschlossen waren, bei 25°C für die angegebene Zeitdauer gelagert. Die Bestimmung der biologischen Aktivität (in vitro) erfolgte, wie beschrieben bei Stewart, W.E. II (1981): The Interferon System (Second, enlarged edition) Springer-Verlag: Wien, New York; Grossberg, S.E. et al. (1984) Assay of Interferons. in: Came, P.E., Carter W.A. (eds.) Interferons and their Applications, Springer-Verlag: Berlin, Heidelberg, New York, Tokyo, pp. 23-43.

Die Ergebnisse sind in den Tabellen 1 bis 5 dargestellt. Bei "% (Ref)" handelt es sich um die Angabe der biologischen Aktivität bezogen auf die biologische Aktivität einer bei -20°C für den angegebenen Zeitraum gelagerten Referenzprobe. Bei "% (OMo)" handelt es sich um die prozentuale biologische Aktivität bezogen auf den Ausgangswert bei 0 Monaten.

**Tabelle 1**

| (Formulierung 1): | | | | |
|---|---|---|---|---|
| Monate | Wirkstoffgehalt | | | |
| | MU/mL | | Recovery (25°C) | |
| | **-20°C** | **25°C** | **% (Ref.)** | **% (OMo.)** |
| 0 | 11,0 | 11,0 | 100 | 100 |
| 1 | 10,0 | 9,8 | 98 | 89 |
| 2 | 9,7 | 11,0 | 113 | 100 |
| 3 | 10,0 | 10,6 | 106 | 96 |
| 4 | 10,3 | 9,5 | 92 | 86 |
| 5 | 9,5 | 9,7 | 102 | 88 |
| 6 | 10,5 | 10,2 | 97 | 93 |

**Tabelle 2**

| (Formulierung 2): | | | | |
|---|---|---|---|---|
| Monate | Wirkstoffgehalt | | | |
| | MU/mL | | Recovery (25°C) | |
| | **-20°C** | **25°C** | **% (Ref.)** | **% (0Mo.)** |
| 0 | 13,9 | 13,9 | 100 | 100 |
| 1 | 14,0 | 11,9 | 85 | 86 |
| 2 | 13,0 | 11,6 | 89 | 83 |
| 3 | 13,1 | 9,6 | 73 | 69 |
| 4 | 12,5 | 8,8 | 70 | 63 |
| 5 | 11,0 | 8,2 | 75 | 59 |
| 6 | 13,3 | 8,4 | 63 | 60 |

**Tabelle 3**

| (Formulierung 3): | | | | |
|---|---|---|---|---|
| Monate | Wirkstoffgehalt | | | |
| | MU/mL | | Recovery (25°C) | |
| | **-20°C** | **25°C** | **% (Ref.)** | **% (0Mo.)** |
| 0 | 12,5 | 12,5 | 100 | 100 |
| 1 | 9,4 | 10,0 | 106 | 80 |
| 2 | 8,3 | 11,5 | 139 | 92 |
| 3 | 7,8 | 11,8 | 151 | 94,4 |
| 4 | 6,8 | 10,3 | 151 | 82,4 |
| 5 | 6,6 | 11,2 | 170 | 89,6 |
| 6 | 7,8 | 13,4 | 172 | 107,2 |

**Tabelle 4**

| (Formulierung 4): | | | | |
|---|---|---|---|---|
| Monate | Wirkstoffgehalt | | | |
| | MU/mL | | Recovery (25°C) | |
| | **-20°C** | **25°C** | **% (Ref.)** | **% (0Mo.)** |
| 0 | 11,4 | 11,4 | 100 | 100 |
| 1 | 10,5 | 10,2 | 97 | 89 |
| 2 | 11,9 | 11,1 | 93 | 97 |
| 3 | 10,8 | 10,0 | 93 | 88 |
| 4 | 10,4 | 9,3 | 89 | 82 |
| 5 | 11,6 | 8,4 | 72 | 74 |
| 6 | 12,4 | 9,5 | 77 | 83 |

**Tabelle 5**

| (Formulierung 5): | | | | |
|---|---|---|---|---|
| Monate | Wirkstoffgehalt | | | |
| | MU/mL | | Recovery (25°C) | |
| | **-20°C** | **25°C** | **% (Ref.)** | **% (0Mo.)** |
| 0 | 11,3 | 11,3 | 100 | 100 |
| 1 | 11,0 | 9,7 | 88 | 86 |
| 2 | 11,7 | 10,1 | 86 | 89 |
| 3 | 11,1 | 10,2 | 92 | 90 |
| 4 | 11,3 | 10,2 | 90 | 90 |
| 5 | 12,0 | 9,2 | 77 | 81 |
| 6 | 11,0 | 9,7 | 88 | 86 |

Aus den obigen Tabellen ist ersichtlich, daß Formulierungen, die kein Humanserumalbumin enthalten (Formulierungen 1,3,4,5), überraschenderweise eine bessere Stabilität als eine Humanserumalbumin enthaltende Formulierung (Formulierung 2) aufweisen.

Bei Formulierung 17 (s.o.) wurde eine Interferonlösung ohne Humanserumalbumin unter aseptischen Bedingungen auf eine Aktivität von 6 MU/0,5 ml eingestellt. Die farblose, klare Lösung wurde anschließend sterilfiltriert und zu je 0,5 ml in vorsterilisierten Einmalspritzen abgefüllt und verschlossen. Die Fertigspritzen wurden bei 25°C gelagert und auf Klarheit, pH-Wert sowie biologische Aktivität untersucht. Dabei ergaben sich folgende Resultate:

| Lagerung in Monaten | pH-Wert | Klarheit [%] | MU/Spritze | | Recovery (25°C) | |
|---|---|---|---|---|---|---|
| | | | -20°C | 25°C | %(Ref.) | %(0Mo.) |
| 0 | 6,5 | 99,5 | 6,3 | 6,3 | 100 | 100 |
| 3 | 6,5 | 99,1 | 5,6 | 6,1 | 108 | 97 |

### 2. Langzeitstabilität von flüssigen IFN-β Formulierungen bei 37°C

Es wurden folgende Formulierungen in Fertigspritzen getestet:
- Formulierung 6:: 50 mmol/l Natriumcitrat, 50 mmol/l Natriumphosphat pH 7,0, 2 mmol/l Methionin
- Formulierung 7:: 50 mmol/l Natriumcitrat pH 5,0, 18 mg/ml Glycerin, 2 mmol/l Methionin
- Formulierung 8:: 50 mmol/l Natriumcitrat pH 5,0, 18 mg/ml Glycerin, 15 mg/ml Humanserumalbumin, 2 mmol/l Methionin
- Formulierung 9:: 50 mmol/l Natriumcitrat pH 6,0, 18 mg/ml Glycerin, 2 mmol/l Methionin
- Formulierung 10:: 50 mmol/l Natriumcitrat pH 6,5, 18 mg/ml Glycerin, 2 mmol/l Methionin

Die Formulierungen wurden in Dosisstärken von 3 MU pro 0,5 ml (Dosisstärke 3), 6 MU pro 0,5 ml (Dosisstärke 6) und 12 MU pro ml (Dosisstärke 12) getestet.

Die Ergebnisse sind in der nachfolgenden Tabelle 6 dargestellt.

**Tabelle 6**

| Lagerung in Monaten | Dosisstärke 3 | | | | | Dosisstärke 6 | | | | | Dosisstärke 12 | | | | |
|---|---|---|---|---|---|---|---|---|---|---|---|---|---|---|---|
| | Formulierung | | | | | Formulierung | | | | | Formulierung | | | | |
| | 6 | 7 | 8 | 9 | 10 | 6 | 7 | 8 | 9 | 10 | 6 | 7 | 8 | 9 | 10 |
| 0 | 100 | 100 | 100 | 100 | 100 | 100 | 100 | 100 | 100 | 100 | 100 | 100 | 100 | 100 | 100 |
| 1 | 71 | 80 | 61 | 74 | 69 | 72 | 85 | 63 | 86 | 84 | 87 | 88 | 71 | 76 | 84 |
| 2 | 51 | 82 | 33 | 74 | 85 | 61 | 81 | 43 | 80 | 76 | 69 | 88 | 48 | 77 | 81 |
| 3 | 44 | 76 | 23 | 63 | 65 | 48 | 64 | 36 | 73 | 69 | 66 | 72 | 35 | 80 | 81 |
| 4 | 33 | 51 | 16 | 61 | 61 | 46 | 65 | 26 | 84 | - | - | 64 | 24 | 78 | 79 |

Aus den Ergenissen von Tabelle 6 ist ersichtlich, daß die erfindungsgemäßen Formulierungen ohne Humanserumalbumin überraschenderweise eine verbesserte Stabilität bei 37°C aufweisen.

### 3. Chemische Stabilität bei 25°C

Um die chemische Stabilität flüssiger Formulierungen von IFN-β zu untersuchen, wurden 7 Ansätze formuliert und bei 25°C eingelagert. Nach 3 bzw. 6 Monaten erfolgte eine Charakterisierung des Proteins mittels eines Lys-C-Mappings und einer kompletten Kohlenhydratanalytik. Ein spezielles Augenmerk wurde auf die Bildung von Methionin-Sulfoxid und die Desialyierung gelegt.

Außer Formulierung 10 (s.o.) wurden folgende Formulierungen getestet:
- Formulierung 11:: 50 mmol/l Natriumcitrat, 50 mmol/l Natriumphosphat, 2 mmol/l Methionin pH 7,0 bis 7,2
- Formulierung 12:: 50 mmol/l Natriumcitrat, 50 mmol/l Natriumphosphat pH 7,0 bis 7,2
- Formulierung 13:: 50 mmol/l Natriumcitrat, 18 mg/ml Glycerin, 2 mmol/l Methionin, pH 5,0 bis 5,2
- Formulierung 14:: 50 mmol/l Natriumcitrat, 18 mg/ml Glycerin, pH 5,0 bis 5,2
- Formulierung 15:: 50mmol/l Natriumcitrat, 15 mg/ml Humanserumalbumin (Medical Grade), 18 mg/ml Glycerin, 2 mmol/l Methionin, pH 5,0 bis 5,2
- Formulierung 16:: 50mmol/l Natriumcitrat, 15 mg/ml Humanserumalbumin (Medical Grade), 18 mg/ml Glycerin, pH 5,0 bis 5,2 (Vergleich)

Der Gehalt an IFN-β lag bei allen Ansätzen zwischen 10 und 11 MU/ml.

### Testdurchführung

Für die Durchführung der Analytik war eine Aufkonzentrierung der Proben notwendig. Zudem mußte bei den Ansätzen 15 und 16 das Humanserumalbumin entfernt werden. Deshalb wurden die Ansätze über eine Anti-β-Chromatographiesäule gegeben. Das Ausgangsvolumen pro Ansatz betrug 32 ml. Die Ansätze 13 bis 16 wurden durch Zugabe von 2,1 ml 0,4 mol/l Na₂HPO₄ und 2,1 ml 0,4 mol/l Na₃PO₄ vor der Anti-β-Chromatographie neutralisiert.

Für die Immunadsorption von Interferon-β an einem monoklonalen Antikörper gegen Interferon-β (B02 Sepharose 6B, cross linked von der Firma Celltech) wurde eine Chromatographiesäule C10 (Firma Pharmacia) mit 5 ml B02-Sepharose gepackt und 3 mal mit je 5-10 Gelvolumina PBS, 0,1 mol/l Natriumphosphat pH 2,0 und PBS/1 mol/l KCl, mit einer linearen Flußrate von 1,0 cm/min gespült.

Der Auftrag von ca. 32 ml der Interferon/HSA haltigen Lösung erfolgte mit einer linearen Flußrate von 0,5 cm/min.

Die Waschung erfolgte mit 10 Gelvolumina PBS/1mol/l KCl mit einer linearen Flußrate von 1 cm/min bis zum Abfall der OD auf die Grundlinie. Die Elution erfolgte mit ca. 1-2 Gelvolumina 0,1 mol/l Natriumphosphat pH 2,0 mit einer linearen Flußrate von 1 cm/min. Interferon-β wird dabei als einzelner Peak in hoher Reinheit erhalten. Dieses Eluat ist für die sich anschließende Proteincharakterisierung geeignet.

### Durchführung der Analytik

### 1. Lys-C-Mapping

Mit dem Enzym Endoproteinase Lys-C aus Achromobacter (AP) wird Interferon-β unter reduzierenden Bedingungen am C-terminalen Ende von Lysin in 12 Peptide gespalten.

In ein Eppendorf-Reaktionsgefäß wurden 50 µl Eluat aus der Anti-β-Chromatographie (12,5-50 µg Interferon-β) gegeben und mit 5 µl 2 mol/l TRIS versetzt. Dazu wurde Endoproteinase der Firma Wako in einem Enzym/Substratverhältnis von 1:10 zugegeben (Endoproteinase Lys-C-Lösung in 50 mmol/l TRIS/HCl, pH 9,0) Die Lösung wurde gemischt und bei 30°C 2 Stunden inkubiert. Danach erfolgte eine Zugabe von 5 µl 0,1 mol/l DTT zum Ansatz.

Die Auftrennung der Peptide erfolgte über eine Reversed Phase Säule (Vydac C18, 300 Å, 5 µm, 2,1 mm) an einer HPLC-Anlage HP 1090 M-Serie mit Diodenarraydetektor bei 214 mm, wobei ein Gradient aus A: 0,1 % (v/v) TFA und B: 0,1% (v/v) TFA/70% (v/v) Acetonitril verwendet wurde. Die Peptide wurden in der Reihenfolge ihrer Retentionszeiten durchnummeriert und sind folgenden Sequenzen zugeordnet.

| **SEQ.ID. NO** | **Peptid** | **Position** | **Sequenz** |
|---|---|---|---|
| 1 | AP1 | 109-115 | EDFTRGK |
| 2 | AP2 | 100-105 | TVLEEK |
| 3 | AP3 | 46-52 | QLQQFQK |
| 4 | AP4(ox) | 116-123 | LM(ox)SSLHLK |
| 5 | AP4 | 116-123 | LMSSLHLK |
| 6 | AP6(ox) | 34-45 | DRM(ox)NFDIPEEIK |
| 7 | AP5 | 124-134 | RYYGRILHYLK |
| 8 | AP6 | 34-45 | DRMNFDIPEEIK |
| 9 | AP7 | 20-33 | LLWQLNGRLEYCLK |
| 10 | AP8(ox) | 1-19 | M(ox)SYNLLGFLQRSSNFQCQK |
| 11 | AP8 | 1-19 | MSYNLLGFLQRSSNFQCQK |
| 12 | AP9 | 137-166 | EYSHCAWTIVRVEILRNFYFINRLTGYLRN |
| 13 | AP10(ox) | 53-99 | |
| 14 | AP10 | 53-99 | |

### Literatur:

Utsumi et al. (1989). Characterization of four different mammalian-cellderived recombinant human interferon-β1. Eur. J. Biochem. 181, 545-553. Utsumi et al. (1988): Structural characterization of fibroblast human interferon-β1. J. Interferon Res. 8, 375-384 Allen, G. (1981): Laboraory techniques in biochemistriy and molecular biology. Sequencing of proteins and peptides. Elsevier Verlag. Castagnola et al. (1988): HPLC in Protein sequence determinations. J. Chromatography 440, 213-251.

In den mit (ox) bezeichneten Peptiden liegt die Aminosäure Methionin als Methioninsulfoxid vor. Die Quantifizierung beruht auf der Bestimmung des Anteils der Peakfläche des oxidierten Peptides zur Gesamtfläche aus intaktem Peptid und oxidiertem Peptid. Die Anteile an oxidierten Methioninen sind in frischen Präparationen von Interferon-β sehr gering. Während der Lagerung nimmt dieser Anteil je nach Lagerbedingungen (Puffer, pH-Wert, Temperatur etc.) mehr oder weniger stark zu. Diese Veränderung ist nicht gewünscht, da sie zur Instabilität des Interferon-β-Moleküls beiträgt bzw. die in vivo Eigenschaften signifikant beeinflussen kann.

Der Anteil der oxidierten Peptide AP4(ox), AP6(ox), AP8(ox) und AP10(ox) ist somit ein wichtiges Kriterium zur Bewertung der chemischen Integrität des Interferon-β-Moleküls in einer flüssigen Formulierung.

### 2. Kohlenhydratbestimmung

Im ersten Schritt wurden die Oligosaccharide vom Polypeptid abgetrennt und entsalzt.

Etwa 0,7 ml des Eluats der Anti-β-Chromatographie wurden in einem Dialyseschlauch (6 mm Duchmesser Sigma No. D-9277) gegen 500 ml Dialysepuffer (0,05 mol/l Natriumphosphat, 0,10 mol/l NaCl, pH 7,25) unter leichtem Rühren 16-20 Stunden bei Raumtemperatur dialysiert. Danach wurde der Schlauch an einem Ende aufgeschnitten und der Inhalt in ein Eppendorf-Reaktionsgefäß gestreift. Das Probevolumen betrug nach der Dialyse 1 ml.

Zu der dialysierten Probe wurden 20 µl Tween 20 (10%ig) und 15 µl N-Glycosidase-F-Lösung (Boehringer Mannheim) pipettiert. Dieses Gemisch wurde 24 Stunden bei 37°C inkubiert. Nach Abschluß der Inkubation wurde bei 10.000 U/min 10 min zentrifugiert, über 0,45 µm filtiert und anschließend über eine Entsalzungssäule (HR 10/10 Pharmacia No. 17-0591-01) mit einem isokratischen Gradienten (Eluent A: destilliertes Wasser) mit einem Fluß von 1,0 ml/min chromatographiert und fraktioniert. Die Detektion der freien Oligosaccharide erfolgte bei 206 nm.

Im zweiten Schritt wurden die freigesetzten Oligosaccharide über einen lonenaustauscher aufgetrennt nach der Anzahl ihrer Sialinsäurenreste differenziert.

Die im Eluat der Entsalzungssäule, ca. 2 ml, enthaltenen Oligosaccharide wurden an einen Anionenaustauscher (Mono Q HR 5/5, Pharmacia No. 17-0546-01) gebunden. Die Asialoformen finden sich im Durchlauf. Mit Hilfe eines flachen NaCl-Gradienten eluierten Monosialo-, Disialo- und Trisialoformen in der angegebenen Reihenfolge deutlich getrennt nacheinander.

| | |
|---|---|
| Eluent A | Milli-Q-Wasser |
| Eluent B | 0,10 mol/l NaCl |

| Gradient | | |
|---|---|---|
| 0 min | 100% A | 0% B |
| 5 min | 100% A | 0% B |
| 25 min | 33% A | 67% B |
| 26 min | 100% A | 0% B |

| | |
|---|---|
| Fluß | 0,75 ml/min |
| Laufzeit | 26 min (mit Regeneration 36 min) |
| Detektion | UV 206 nm |

Die Detektion der einzelnen Oligosaccharidfraktionen erfolgte mittels eines UV-Detektors bei 206 nm. Die quantitative Berechnung wurde über die Integration der Flächen der einzelnen Peaks durchgeführt.

Die Oligosaccharidfraktionen Monosialo, Disialo und Trisialo wurden anschließend, wie oben beschrieben, über eine Entsalzungssäule geleitet.

Im dritten Schritt werden die geladenen Oligosaccharide in neutrale Oligosaccharide überführt, indem unter sauren pH-Bedingungen die endständigen Sialinsäurenreste hydrolytisch abgespalten wurden.

Dazu wurden von jeder Oligosaccharidfraktion ca. 15 µl plus 15 µl Milli Q Wasser in ein Mikroteströhrchen gegeben und 30 µl 10 mmol/l H₂SO₄ zugefügt. Anschließend wurde 90 min lang auf 80°C erhitzt.

Danach wurde 1 min bei 5000 U/min zentrifugiert und der Ansatz in ein Minivial pipettiert. Die jetzt neutralen Kohlenhydrate werden bei alkalischem pH-Wert zu schwachen Anionen und an eine Anionenaustauschersäule (CarboPac PA1 (4x250 mm) P/N 35391, Dionex) gebunden. Die Elution erfolgt mit einem Gradienten aus

| | |
|---|---|
| Eluent A | NaOH 0,16 mol/l |
| Eluent B | NaOH 0,16 mol/l Na-Acetat 0,10 mol/l |
| Eluent C | NaOH 0,16 mol/l Na-Acetat 0,75 mol/l |

| Gradient: | | | |
|---|---|---|---|
| 0 min | 95% A | 5% B | 0% C |
| 2,0 min | 95% A | 5% B | 0% C |
| 3,0 min | 85% A | 15% B | 0% C |
| 4,0 min | 85% A | 15% B | 0% C |
| 28,0 min | 37% A | 63% B | 0% C |
| 28,1 min | 90% A | 0% B | 10% C |
| 45,0 min | 20% A | 0% B | 80% C |
| 45,1 min | 95% A | 5% B | 0% C |
| 50,0min | 95% A | 5% B | 0% C |

| | |
|---|---|
| Fluß | 1,0 ml/min |
| Laufzeit | 50 min |
| Detektion | PAD |

Zur Bestimmung der Oligosaccharide wird die PAD (Pulsed Amperometric Detection) verwendet. Das Oligosaccharidmolekül wird elektrochemisch oxidiert und der dabei entstehende Strom gemessen. Die PAD zeichnet sich durch eine hohe Empfindlichkeit aus, so daß ein Nachweis im ng-Bereich ohne Schwierigkeiten möglich ist. Das Ausgangssignal am Detektor (in mV) ist der Menge an Kohlenydrat direkt proportional. Die Quantifizierung erfolgt über die Integration der Peakflächen.

Die Proben wurden zwischen der Deglykosilierung und der Analyse bei -20°C zwischengelagert.

### Literatur:

Townsend (1988): High-performance anion-exchange chromatography of oligosaccharides. Analytical Biochemistry 174, 459-470.

### Ergebnisse

### 1. Lys-C-Mapping

Das Lys-C-Mapping der Ansätze 11 bis 16 zeigte hinsichtlich der Retentionszeit und der Qualifizierung der Peptide keinen Unterschied zum Ausgangswert.

Die Bestimmung des Gehalts von Methioninsulfoxid während der Flüssiglagerung ergab die in den Tabellen 7 (Lagerung für 3 Monate) und 8 (Lagerung für 6 Monate) gezeigten Ergebnisse.

**Tabelle 7**

| Bezeichnung | Anteil AP4ox | Anteil AP6ox | Anteil AP8ox | Anteil AP10ox |
|---|---|---|---|---|
| to-Wert | < 5% | 7,6% | LOD | LOD |
| Formulierung 11 | 7,9% | 10,5% | LOD | LOD |
| Formulierung 12 | < 5% | 11,6% | LOD | LOD |
| Formulierung 13 | < 5% | 7,3% | LOD | LOD |
| Formulierung 14 | < 5% | 9,4% | LOD | LOD |
| Formulierung 15 | < 5% | 8,6% | LOD | LOD |
| Formulierung 16 | < 5% | 10,8% | LOD | LOD |
| (LOD = nicht nachweisbar) | | | | |

**Tabelle 8**

| Bezeichnung | Anteil AP4ox | Anteil AP6ox | Anteil AP8ox | Anteil AP10ox |
|---|---|---|---|---|
| to-Wert | < 5% | 7,6% | LOD | LOD |
| Formulierung 10 | 7,6% | 8,9% | LOD | LOD |
| Formulierung 11 | 7,7% | 9,5% | LOD | LOD |
| Formulierung 12 | 12,0% | 13,7% | LOD | LOD |
| Formulierung 13 | 7,4% | 8,7% | LOD | LOD |
| Formulierung 14 | 13,7% | 15,7% | LOD | LOD |
| Formulierung 15 | 7,4% | 7,9% | LOD | LOD |
| Formulierung 16 | 18,0% | 17,6% | LOD | LOD |

Aus Tabelle 7 ist ersichtlich, daß bei einer dreimonatigen Lagerung die methioninhaltigen Ansätze 13 und 15 gegenüber den methioninfreien Ansätzen einen geringeren Anteil an Methioninsulfoxid zeigen. Nach einer sechsmonatigen Lagerung wird der Einfluß des zugesetzten Methionins in den Ansätzen 11, 13 und 15 deutlicher. Dort ist nur eine sehr geringe Zunahme des Gehalts an Methioninsulfoxid nachweisbar. In den methioninfreien Ansätzen nimmt der Gehalt an Methioninsulfoxid etwas stärker zu, liegt aber in der Summe aller oxidierten Methioninanteile zum Gesamtgehalt an Methionin unter 10%.

### 2. Kohlenhydratbestimmung

In den Tabellen 9a, 9b, 10a, 10b, 11a und 11b sind die Ergebnisse der Kohlenhydratbestimmung nach drei bzw. nach 6 Monaten Lagerzeit angegeben.

Interferon-β-1a besitzt an seiner Aminosäurenkette eine Kohlenhydratstruktur, die sich aus einer definierten Reihenfolge von Monosacchariden aufbaut. Je nach Verzweigungsart spricht man von biantennären (2 Arme), triantennären (3 Arme) und tetraantennären (4 Arme) Strukturen.

Die Kohlenhydratstruktur baut sich aus den Monosacchariden Mannose, Fucose, N-Acetylglucosamin, Galactose und Sialinsäure auf.

Dabei nimmt die Sialinsäure in mehrfacher Hinsicht eine Sonderstellung ein:
- Sie ist das einzige Monosaccharid mit einer geladenen Gruppe (Carboxylgruppe).
- Sie tritt immer endständig in der Kohlenhydratkette auf.
- Sie ist enzymatisch bzw. hydrolytisch wesentlich leichter abspaltbar als die restlichen Monosaccharide.
- Während die neutrale Kohlenhydratkette in ihrer Struktur sehr konstant ist, treten beim Anteil der Sialinsäure große Schwankungen auf, die unter anderem von der Zellkultur und dem Aufreinigungsverfahren des Interferons abhängig sind.

### Literatur:

Kagawa et al., J. Biol. Chem. 263 (1988), 17508-17515; EP-A-0 529 300.

Es wurde der Sialostatus (prozentualer Anteil einzelner Sialostrukturen) nach dreimonatiger Lagerung (Tabelle 9a) bzw. sechsmonatiger Lagerung (Tabelle 9b) untersucht. Eine Kohlenhydratstruktur, die endständig keine Sialinsäure enthält, wird als Asialo bezeichnet. Eine Kohlenhydratstruktur die endständig eine Sialinsäure enthält, wird als Monosialo bezeichnet. Eine Kohlenhydratstruktur, die endständig zwei Sialinsäuren enthält, wird als Disialo bezeichnet. Eine Kohlenhydratstruktur, die endständig drei Sialinsäuren enthält, wird als Trisialo bezeichnet.

Weiterhin wurde die Antennärität (prozentualer Anteil einzelner Verzweigungsarten) nach dreimonatiger Lagerung (Tabelle 10a) bzw. nach sechsmonatiger Lagerung (Tabelle 10b) bestimmt. Eine Kohlenhydratstruktur mit einer Verzweigung und damit zwei endständigen Galactosen wird als biantennär bezeichnet. Sie kann terminal mit null bis zwei Sialinsäuren besetzt sein. Eine Kohlenhydratstruktur mit zwei Verzweigungen und damit drei endständigen Galactosen wird als triantennär bezeichnet. Sie kann terminal mit null bis drei Sialinsäuren besetzt sein.

Außerdem wurde der Sialylierungsgrad (prozentuale Belegung terminaler Galactosereste mit Sialinsäure) nach dreimonatiger Lagerung (Tabelle 11a) bzw. nach sechsmonatiger Lagerung (Tabelle 11b) untersucht.

Aus den Ergebnissen ist ersichtlich, daß durch die Lagerung bei pH 5 eine geringe, aber reproduzierbare Desialylierung stattfindet. Eine Lagerung bei pH 7 beeinflußt den Sialylierungsgrad nicht.

Der in den Ansätzen 15 und 16 angegebene afuco-Anteil stammt vermutlich von Fremdproteinen aus dem zugesetzten Humanserumalbumin, die durch die Anti-β-Chromatographie nicht quantitativ abgetrennt wurden.

Bezüglich der Antennärität erfolgt kein meßbarer Einfluß durch die Flüssiglagerung.

**Tabelle 9a**

| Bezeichnung | Asialo | Monoasialo | Disialo | Trisialo |
|---|---|---|---|---|
| to-Wert | < 3 | 13,4 | 73,4 | 12,1 |
| Formulierung 11 | < 3 | 14,0 | 74,1 | 11,9 |
| Formulierung 12 | < 3 | 12,6 | 74,9 | 11,6 |
| Formulierung 13 | < 3 | 16,5 | 70,4 | 12,0 |
| Formulierung 14 | < 3 | 16,6 | 71,1 | 11,1 |
| Formulierung 15 | < 3 | 15,8 | 70,0 | 13,0 |
| Formulierung 16 | < 3 | 15,1 | 72,0 | 11,9 |

**Tabelle 9b**

| Bezeichnung | Asialo | Monosialo | Disialo | Trisialo |
|---|---|---|---|---|
| to-Wert | < 3 | 13,4 | 73,4 | 12,1 |
| Formulierung 10 | < 3 | 13,9 | 70,2 | 15,3 |
| Formulierung 11 | < 3 | 14,5 | 73,9 | 11,6 |
| Formulierung 12 | < 3 | 14,0 | 72,4 | 13,6 |
| Formulierung 13 | < 3 | 18,6 | 68,9 | 11,7 |
| Formulierung 14 | < 3 | 19,0 | 69,4 | 10,7 |
| Formulierung 15 | < 3 | 17,0 | 71,0 | 11,3 |
| Formulierung 16 | < 3 | 16,1 | 71,5 | 12,4 |

**Tabelle 10a**

| Bezeichnung | Biantennär | Triantennär 1->6 | Triantennär + 1 repeat |
|---|---|---|---|
| to-Wert | 74,4 | 18,1 | 3,7 |
| Formulierung 11 | 72,9 | 18,7 | 3,7 |
| Formulierung 12 | 76,9 | 17,0 | 2,7 |
| Formulierung 13 | 74,7 | 18,0 | 3,1 |
| Formulierung 14 | 75,9 | 17,3 | 2,9 |
| Formulierung 15 | 76,2 (inkl. 5% afuco) | 18,0 | 3,3 |
| Formulierung 16 | 76,9 (inkl. 5% afuco) | 17,8 | 3,0 |

**Tabelle 10b**

| Bezeichnung | Biantennär | Triantennär 1->6 | Triantennär+ 1 repeat |
|---|---|---|---|
| to-Wert | 74,4 | 18,1 | 3,7 |
| Formulierung 10 | 71,4 | 19,3 | 4,0 |
| Formulierung 11 | 73,0 | 18,7 | 3,3 |
| Formulierung 12 | 72,3 | 19,7 | 3,4 |
| Formulierung 13 | 72,4 | 19,2 | 3,4 |
| Formulierung 14 | 74,2 | 18,7 | 3,2 |
| Formulierung 15 | 73,0 | 18,7 | 2,8 |
| Formulierung 16 | 74,3 (inkl. 4% afuco) | 19,7 | 3,2 |

**Tabelle 11a**

| Bezeichnung | Sialylierungsgrad |
|---|---|
| to-Wert | 88,3 |
| Formulierung 11 | 87,0 |
| Formulierung 12 | 88,2 |
| Formulierung 13 | 85,8 |
| Formulierung 14 | 85,8 |
| Formulierung 15 | 86,6 |
| Formulierung 16 | 86,9 |

**Tabelle 11b**

| Bezeichnung | Sialylierungsgrad |
|---|---|
| to-Wert | 88,3 |
| Formulierung 10 | 87,5 |
| Formulierung 11 | 86,6 |
| Formulierung 12 | 87,7 |
| Formulierung 13 | 84,1 |
| Formulierung 14 | 84,3 |
| Formulierung 15 | 85,7 |
| Formulierung 16 | 86,5 |

## Patentansprüche

1. Flüssige Formulierung, die humanes glykosiliertes Interferon-β als Wirkstoff, einen Puffer zur Einstellung eines pH-Wertes von 5 bis 8 und Methionin enthält, die frei von Humanserumalbumin ist und nach Lagerung für 3 Monate bei 25 °C eine Langzeitstabilität der biologischen *in vitro* Aktivität von mindestens 80 % der Ausgangsaktivität aufweist, mit der Maßgabe, dass die Formulierung keine sauren Aminosäuren, Arginin oder Glycin in einem Gewichtsanteil zwischen 0,3 bis 5 % enthält.

2. Formulierung nach Anspruch 1 oder 2,
**dadurch gekennzeichnet,**
**dass** das Interferon-β aus CHO-Zellen stammt.

3. Formulierung nach einem der Ansprüche 1 bis 2,
**dadurch gekennzeichnet,**
**dass** sie den Puffer in einer Konzentration von 10 mmol/l bis 1 mol/l enthält.

4. Formulierung nach einem der Ansprüche 1 bis 3,
**dadurch gekennzeichnet,**
**dass** sie einen Puffer ausgewählt aus der Gruppe bestehend aus Phosphat-, Citrat- und Acetatpuffern und Mischungen davon enthält.

5. Formulierung nach Anspruch 4,
**dadurch gekennzeichnet,**
**dass** sie einen Phosphat/Citratpuffer enthält.

6. Formulierung nach einem der Ansprüche 1 bis 5,
**dadurch gekennzeichnet,**
**dass** sie einen pH-Wert zwischen 6 und 7,2 aufweist.

7. Formulierung nach einem der Ansprüche 1 bis 6,
**dadurch gekennzeichnet,**
**dass** sie abgesehen vom Wirkstoff frei von humanen oder tierischen Polypeptiden ist.

8. Formulierung nach einem der Ansprüche 1 bis 7,
**dadurch gekennzeichnet,**
**dass** sie frei von oberflächenaktiven Verbindungen ist.

9. Formulierung nach einem der Ansprüche 1 bis 8,
**dadurch gekennzeichnet,**
**dass** sie eine chemische Integrität nach Lagerung bei 25 °C für 6 Monate aufweist.

10. Formulierung nach einem der Ansprüche 1 bis 9,
**dadurch gekennzeichnet,**
**dass** sie eine physikalische Integrität nach Lagerung bei 25 °C für 6 Monate aufweist.

11. Formulierung nach Anspruch 1,
**dadurch gekennzeichnet,**
**dass** das Methionin in einer Konzentration von 0,1 bis 4 mmol/l vorliegt.

12. Formulierung nach einem der Ansprüche 1 bis 11,
**dadurch gekennzeichnet,**
**dass** sie weiterhin Hilfsstoffe zur Einstellung der Tonizität enthält.

13. Formulierung nach einem der Ansprüche 1 bis 12,
**dadurch gekennzeichnet,**
**dass** sie weiterhin Verdickungsmittel zur Viskositätserhöhung **enthält.**

14. Formulierung nach einem der Ansprüche 1 bis 13,
**dadurch gekennzeichnet,**
**dass** sie weiterhin physiologisch verträgliche Konservierungsmittel enthält.

15. Pharmazeutisches Präparat,
**dadurch gekennzeichnet,**
**dass** es eine flüssige Formulierung nach einem der Ansprüche 1 bis 14 enthält.

16. Pharmazeutisches Präparat nach Anspruch 15 zur oralen, parenteralen oder ophthalmologischen Applikation.

17. Pharmazeutisches Präparat nach Anspruch 15 oder 16 in Form von Einzeldosen von 1 bis 25 MU.

18. Verfahren zur Herstellung eines pharmazeutischen Präparats nach einem der Ansprüche 15 bis 17,
**dadurch gekennzeichnet,**
**dass** man eine Formulierung nach einem der Ansprüche 1 bis 14 und gegebenenfalls weitere galenisch notwendige Hilfsstoffe zubereitet und in eine geeignete Darreichungsform bringt.

19. Verfahren zur Verbesserung der Haltbarkeit einer flüssigen Formulierung, die humanes Interferon-β als Wirkstoff und einen Puffer zur Einstellung eines pH-Wertes von 5 bis 8 enthält,
**dadurch gekennzeichnet,**
**dass** man eine Formulierung ohne Humanserumalbumin und mit Methionin verwendet, mit der Maßgabe, dass die Formulierung keine sauren Aminosäuren, Arginin oder Glycin in einem Gewichtsanteil zwischen 0,3 bis 5 % enthält.

20. Verfahren nach Anspruch 19,
**dadurch gekennzeichnet,**
**dass** die Verbesserung der Haltbarkeit eine Verbesserung der Langzeitstabilität der biologischen Aktivität in vitro, der chemischen Integrität oder/und der physikalischen Integrität umfasst.

## Claims

1. Liquid formulation which contains human glycosylated interferon-β as the active substance, a buffer for adjusting a pH of 5 to 8 and methionine, which is free from human serum albumin and has a long-term stability of the biological *in vitro* activity of at least 80 % of the initial activity after storage for 3 months at 25°C provided that the formulation does not contain acidic amino acids, arginine or glycine in a weight percentage between 0.3 and 5 %.

2. Formulation according to claim 1,
**characterized in that**
the interferon-β is derived from CHO cells.

3. Formulation according to one of the claims 1 to 2,
**characterized in that**
it contains a buffer at a concentration of 10 mmol/l to 1 mol/l.

4. Formulation according to one of the claims 1 to 3,
**characterized in that**
it contains a buffer selected from the group comprising phosphate, citrate and acetate buffers and mixtures thereof.

5. Formulation according to claim 4,
**characterized in that**
it contains a phosphate/citrate buffer.

6. Formulation according to one of the claims 1 to 5,
**characterized in that**
it has a pH between 6 and 7.2.

7. Formulation according to one of the claims 1 to 6,
**characterized in that**
it is free from human or animal polypeptides apart from the active substance.

8. Formulation according to one of the claims 1 to 7,
**characterized in that**
it is free from surface-active compounds.

9. Formulation according to one of the claims 1 to 8,
**characterized in that**
it exhibits a chemical integrity after storage of 6 months at 25°C.

10. Formulation according to one of the claims 1 to 9,
**characterized in that**
it exhibits a physical integrity after storage of 6 months at 25°C.

11. Formulation according to claim 1,
**characterized in that**
the methionine is present at a concentration of 0.1 to 4 mmol/l.

12. Formulation according to one of the claims 1 to 11,
**characterized in that**
it additionally contains auxiliary substances to adjust the tonicity.

13. Formulation according to one of the claims 1 to 12,
**characterized in that**
it additionally contains thickening agents to increase the viscosity.

14. Formulation according to one of the claims 1 to 13,
**characterized in that**
it additionally contains physiologically acceptable preservatives.

15. Pharmaceutical preparation,
**characterized in that**
it contains a liquid formulation according to one of the claims 1 to 14.

16. Pharmaceutical preparation according to claim 15 for oral, parenteral or opthalmological application.

17. Pharmaceutical preparation according to claim 15 or 16 in the form of single doses of 1 to 25 MU.

18. Process for producing a pharmaceutical preparation according to one of the claims 15 to 17,
**characterized in that**
a formulation according to one of the claims 1 to 14 and optionally additional galenically necessary auxiliary substances is prepared and made into a suitable form of administration.

19. Process for improving the storage stability of a liquid formulation which contains human interferon-β as the active substance and a buffer for adjusting a pH of 5 to 8,
**characterized in that**
a formulation without human serum albumin and containing methionine is used provided that the formulation does not contain acidic amino acids, arginine or glycine in a weight percentage between 0.3 and 5 %.

20. Process according to claim 19,
**characterized in that**
the improvement of the storage stability comprises an improvement of the long-term stability of the biological activity in vitro, of the chemical integrity or/and of the physical integrity.

## Revendications

1. Formulation liquide qui contient de l'interféron β humain glycosylé comme principe actif, un tampon pour établir un pH de 5 à 8 et de la méthionine, qui est dépourvue de sérumalbumine humaine et qui, après un stockage de 3 mois à 25°C, présente une stabilité à long terme de l'activité biologique *in vitro* d'au moins 80 % de l'activité initiale, à condition que la formulation ne contienne pas d'acide aminé acide, d'arginine ou de glycine en une proportion massique de 0,3 à 5 %.

2. Formulation selon la revendication 1 **caractérisée en ce que** l'interféron β provient de cellules CHO.

3. Formulation selon l'une des revendications 1 à 2 **caractérisée en ce qu'**elle contient le tampon en une concentration de 10 mmol/l à 1 mol/l.

4. Formulation selon l'une des revendications 1 à 3 **caractérisée en ce qu'**elle contient un tampon choisi dans le groupe consistant en les tampons phosphate, citrate et acétate et leurs mélanges.

5. Formulation selon la revendication 4 **caractérisée en ce qu'**elle contient un tampon phosphate/citrate.

6. Formulation selon l'une des revendications 1 à 5 **caractérisée en ce qu'**elle présente un pH de 6 à 7,2.

7. Formulation selon l'une des revendications 1 à 6 **caractérisée en ce que**, mis à part le principe actif, elle est dépourvue de polypeptides humains ou animaux.

8. Formulation selon l'une des revendications 1 à 7 **caractérisée en ce qu'**elle est dépourvue de composés tensioactifs.

9. Formulation selon l'une des revendications 1 à 8 **caractérisée en ce qu'**elle présente une intégrité chimique après un stockage à 25°C pendant 6 mois.

10. Formulation selon l'une des revendications 1 à 9 **caractérisée en ce qu'**elle présente une intégrité physique après un stockage à 25°C pendant 6 mois.

11. Formulation selon la revendication 1 **caractérisée en ce que** la méthionine est présente en une concentration de 0,1 à 4 mmol/l.

12. Formulation selon l'une des revendications 1 à 11 **caractérisée en ce qu'**elle contient en outre des adjuvants pour régler la pression osmotique.

13. Formulation selon l'une des revendications 1 à 12 **caractérisée en ce qu'**elle contient en outre des épaississants pour augmenter la viscosité.

14. Formulation selon l'une des revendications 1 à 13 **caractérisée en ce qu'**elle contient en outre des conservateurs physiologiquement acceptables.

15. Préparation pharmaceutique **caractérisée en ce qu'**elle contient une formulation liquide selon l'une des revendications 1 à 14.

16. Préparation pharmaceutique selon la revendication 15 pour l'application orale, parentérale ou ophtalmologique.

17. Préparation pharmaceutique selon la revendication 15 ou 16 sous forme de doses unitaires de 1 à 25 MU.

18. Procédé de production d'une préparation pharmaceutique selon l'une des revendications 15 à 17 **caractérisé en ce que** l'on prépare une formulation selon l'une des revendications 1 à 14 et éventuellement d'autres adjuvants nécessaires du point de vue galénique et on les incorpore dans une forme d'administration appropriée.

19. Procédé pour améliorer la durabilité d'une formulation liquide qui contient de l'interféron P comme principe actif et un tampon pour établir un pH de 5 à 8, **caractérisé en ce que** l'on utilise une formulation sans sérumalbumine humaine et avec de la méthionine à condition que la formulation ne contienne pas d'acide aminé acide, d'arginine ou de glycine en une proportion massique de 0,3 à 5 %.

20. Procédé selon la revendication 19 **caractérisé en ce que** l'amélioration de la durabilité comprend une amélioration de la stabilité à long terme de l'activité biologique in vitro, de l'intégrité chimique et/ou de l'intégrité physique.
